# EUROPEAN PATENT APPLICATION

(11) **EP 3 680 655 A1**
(43) Date of publication of application: **15.07.2020**
(21) Application number: 18850855.0
(22) Date of filing: 29.08.2018
(51) Int. Cl.: G01N 31/00, B41F 23/04, B65F 1/14, G01N 27/00, G01N 27/06, H05B 41/292

(54) **HIGH-FREQUENCY-POWER-SOURCE UV LAMP MONITOR, AND TOTAL ORGANIC CARBON METER IN WHICH SAME IS USED**

(30) Priority: 04.09.2017 JP 2017169420
(71) Applicant: Shimadzu Corporation, Kyoto 604-8511 (JP)
(72) Inventor: YAHATA Masahito, Kyoto-shi Kyoto 604-8511 (JP)
(74) Representative: Müller-Boré & Partner Patentanwälte PartG mbB
(86) International application number: PCT/JP2018/031895
(87) International publication number: WO 2019/044881

(57) **Abstract**

Provided is a high-frequency power source UV lamp monitor capable of easily confirming lighting of a UV lamp built in a housing having a hermetically sealed structure. The monitor is attached to a high-frequency power UV lamp 20 that emits ultraviolet light by supplying high-frequency energy from a high-frequency power source 19 via a feeder, and is configured to include a detection circuit having a core member attached around the feeder in a ring shape and made of a magnetically permeable material, an induction circuit wound around the core member to extract a high-frequency induction current, and a detection element connected to the induction circuit.

## Description

### Technical Field

The present invention relates to a UV lamp monitor attached to a UV lamp (ultraviolet lamp) for monitoring the lighting state thereof and a total organic carbon meter (hereinafter referred to as "TOC meter") using the same, and more particularly, to a high-frequency power source UV lamp monitor attached to a high-frequency power source UV lamp for emitting ultraviolet rays using a high-frequency power source, such as, e.g., an excimer lamp, and a TOC meter using the same.

### BACKGROUND OF THE INVENTION

When measuring an organic substance contained in sample water, such as, e.g., wastewater, a TOC meter is used to measure the amount of carbon contained in the organic substance. In the TOC meter using a wet oxidation method, a sample oxidizing portion for oxidizing and decomposing an organic substance by irradiating ultraviolet rays to the sample water to be analyzed is provided. In this oxidizing portion, a low-pressure mercury lamp, which is easily available, is used as a UV lamp for emitting ultraviolet rays having a wavelength shorter than 200 nm.

In a TOC meter using a UV lamp, the total carbon (TC) amount is measured by the amount of carbon dioxide generated from the total carbon (TC) in the sample water by supplying power from a power source and turning on the UV lamp to cause an oxidation reaction in the sample water. At this time, in order to prevent the ultraviolet rays of the sample oxidizing portion from leaking to the outside for the reasons that it is dangerous if the ultraviolet rays are directly irradiated to eyes and it is necessary to prevent generation of harmful ozone due to irradiation of ultraviolet rays to oxygen in the air, or the like, for example, the sample oxidizing portion is configured by a housing having a hermetically sealed structure and the UV lamp is built in the housing (see Patent Document 1).

On the other hand, if the UV lamp is not turned on due to a failure or abnormality even though the power is in a turned-on state during the analysis and measurement, the oxidation reaction is not performed, so that an erroneous measurement result is shown. For this reason, it is necessary to confirm whether or not the UV lamp is turned on in the power-on state before starting the measurement.

In the UV lamp built in the housing having the above-described hermetically sealed structure, however, it is impossible to confirm the lighting state. Therefore, it is disclosed that a lighting confirmation window made of a material that allows transmission of visible light and does not allow transmission of ultraviolet rays is provided in a housing and that a sealed portion (lead wire leading out portion) of a UV lamp tube body in which an ultraviolet radiation gas is sealed is formed of a resin having a property of allowing transmission of visible light while shielding ultraviolet rays and the sealed portion is configured to protrude from the housing so that visible light can be visually recognized from the sealed portion when the UV lamp is turned on (see Patent Document 2).

In addition to the lighting confirmation method described in the above Patent Document, for example, a photosensor for measuring illuminance of a UV lamp is attached as a lamp monitor to confirm lighting in a housing of a sample oxidizing portion in which the UV lamp is built-in (see Patent Document 1).

### Prior Art Document

### Patent Document

Patent Document 1: Japanese Unexamined Patent Application Publication No. 2007-040729
Patent Document 2: Japanese Unexamined Patent Application Publication No. 2010-266222

### SUJ\rllVIARY OF THE INVENTION

### Problems to be Solved by the Invention

The method of forming a window in the housing itself in which a UV lamp is built-in or using a resin that allow transmission of visible light while shielding ultraviolet light in the sealed portion of the UV lamp tube is useful as a method of confirming lighting of a UV lamp. However, in the former method, it is necessary to hermetically seal the window to form a sealed structure, and in the latter case, a UV lamp with special specifications is necessary.

Further, although a method of arranging a UV lamp and a photosensor for lighting confirmation in parallel in a housing having a hermetically sealed structure is also useful, it is necessary to separately provide wiring for a photosensor and a driving power source. Further, in the event of a failure of the photosensor, there is a fear that a signal indicating that the UV lamp is turned off is erroneously transmitted even though the UV lamp is in a normally turned-on state.

On the other hand, in the UV lamp of the TOC meter, it is desirable to use a UV lamp not using mercury instead of a conventional low-pressure mercury lamp in consideration of the environmental aspect of demercuriation, apart from the above-mentioned structural problem. As a UV lamp that can obtain ultraviolet rays of 200 nm or less and does not use mercury, although it is conceivable to use a xenon arc lamp. However, even if demercuriation is achieved by a xenon arc lamp, the problem at the time of confirming the lighting of the UV lamp in the hermetically sealed housing cannot be solved.

Therefore, the present invention aims to provide a high-frequency power source UV lamp monitor capable of easily confirming lighting of a high-frequency power source UV lamp built in a housing having a hermetically sealed structure. The present invention also aims to provide a TOC meter using a high-frequency power source UV lamp monitor capable of achieving demercuriation by a high-frequency power source UV lamp that does not use mercury and performing lighting confirmation by a simple method.

### J\rleans for Solving the Problem

A high-frequency power source UV lamp monitor according to the present invention, which has been made to solve the above-mentioned problems, is a high-frequency power source UV lamp monitor attached to a high-frequency power source UV lamp which emits ultraviolet light by supplying high-frequency energy from a high-frequency power source via a feeder. The high-frequency power source UV lamp monitor includes a detection circuit having a core member attached around the feeder in a ring shape and made of a magnetically permeable material, an induction circuit wound around the core member to extract a high-frequency induction current, and a detection element connected to the induction circuit.

Here, the "high-frequency power source UV lamp" refers to a lamp in which discharge is performed by supplying high-frequency energy having a frequency of 10 kHz or more. More specifically, an excimer lamp, a xenon flash lamp, or the like, which emits ultraviolet light having a wavelength shorter than 200 nm by dielectric barrier discharge, corresponds to the lamp.

The "detection element" may be any element capable of detecting as a signal that a high-frequency induced current has flowed through the induction circuit, and specifically, an LED capable of detecting as a light emission signal is desirable.

Further, a total organic carbon meter according to the present invention, which has been made from another viewpoint, is a total organic carbon meter including a sample oxidizing portion for oxidizing carbon contained in a sample solution by irradiation with ultraviolet rays to convert the carbon into carbon dioxide, wherein the sample oxidizing portion uses a high-frequency power source UV lamp for emitting ultraviolet rays by supplying high-frequency energy from a high-frequency power source via a feeder, and is provided with a high-frequency power source UV lamp monitor for confirming lighting of the high-frequency power source UV lamp, and wherein the high-frequency power source UV lamp monitor is provided with a core member attached around the feeder in a ring shape and made of a magnetically permeable material, an induction circuit wound around the core member for extracting a high-frequency induction current, and a detecting element connected to the induction circuit.

According to the present invention, the high-frequency power source UV lamp is turned on when high-frequency energy is supplied from the high-frequency power source. At this time, some deviation of the impedance matching occurs in the feeder connecting the high-frequency power source and the high-frequency power source UV lamp, so that the common mode current is induced by the influence of the deviation. Although impedance matching can theoretically be mitigated by incorporating a matching circuit, it is difficult to completely cancel out. Further, in the present invention, since it is only necessary to turn on the high-frequency power source UV lamp, it is unnecessary to provide a matching circuit if the impedance matching is not excessively deviated, and it is possible to obtain an induced current by supplying high-frequency energy without performing the matching adjustment.

In this manner, electromagnetic waves are generated around the feeder by the high-frequency energy, and are extracted as a high-frequency induction current by the induction circuit wound around the core member around the feeder. At this time, if a high-frequency induction current is flowing, it can be detected by the detection element connected to the induction circuit, so that the lighting can be confirmed by this detection element.

### Effects of the Invention

According to the high-frequency power source UV lamp monitor of the present invention, the lighting of the high-frequency power source UV lamp can be easily confirmed by detecting the high-frequency induction current generated around the feeder outside the container with the detection element in a state in which the high-frequency power source UV lamp is built in the hermetically sealed container. Therefore, it is possible to confirm the lighting of the lamp in the hermetically sealed container safely without risk of the ultraviolet rays entering eyes of the user and without leakage of harmful ozone.

Further, according to the high-frequency power UV lamp monitor of the present invention, it is possible to confirm lighting without providing wiring for extracting a signal from the inside of the hermetically sealed container to the outside or a photosensor or the like which requires a drive power source separately.

Further, by employing the TOC meter using the high-frequency power source UV lamp monitor of the present invention, the TOC meter can be applied to confirmation of lighting of the high-frequency power source UV lamp used in the sample oxidizing portion of the TOC meter, and moreover, the TOC meter can be provided without using a mercury lamp having a large environmental load.

### BRIEF DESCRIPTION OF THE DRAWINGS

FIG. 1 is a configuration diagram showing a TOC meter using a high-frequency power source UV lamp monitor according to the present invention.
FIG. 2 is a diagram showing a configuration of a sample oxidizing portion of FIG. 1.
FIG. 3A is a wiring diagram of a high-frequency power source UV lamp and a monitor in the sample oxidizing portion of FIG. 1.
FIG. 3B is a wiring diagram showing a modified embodiment of the detector in FIG. 3A.

### ElVIBODilVIENTS FOR CARRYING OUT THE INVENTION

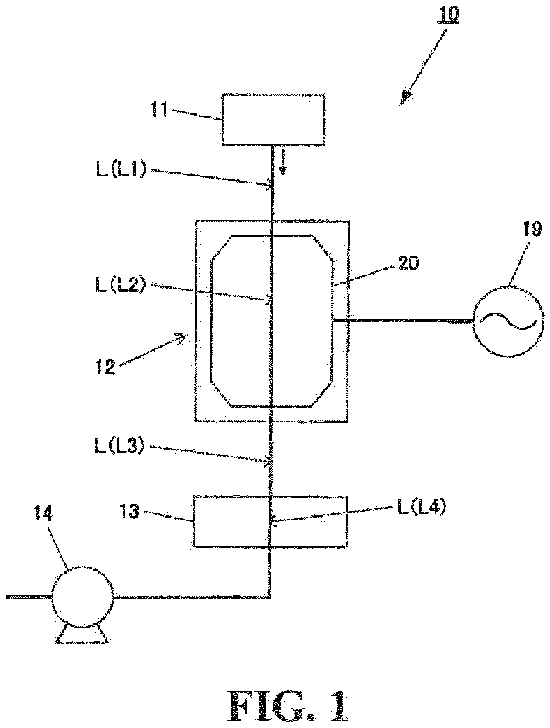

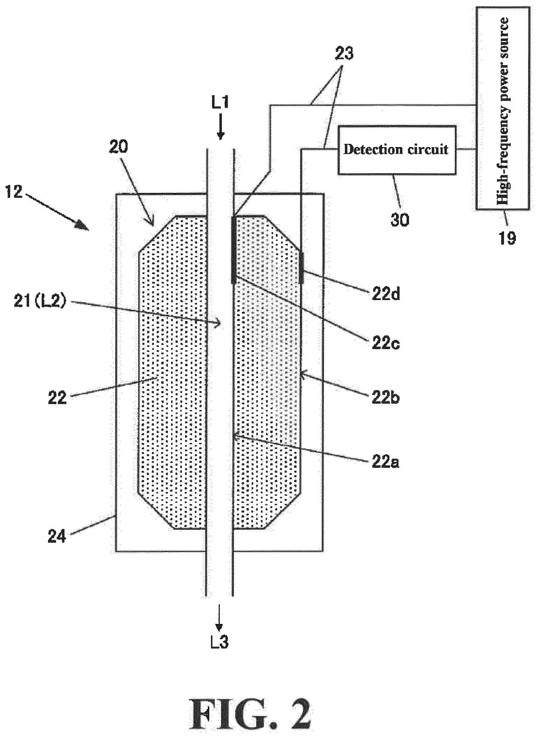

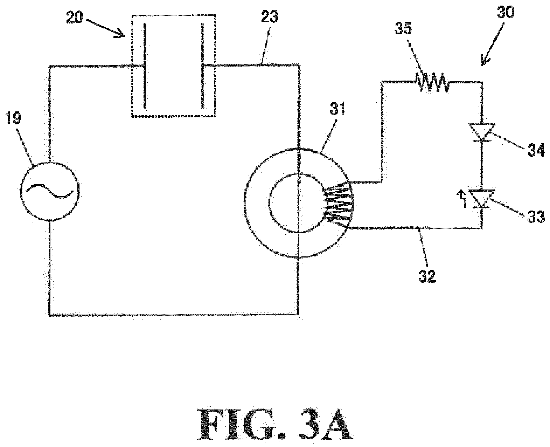

Hereinafter, an embodiment in which a high-frequency power source UV lamp monitor according to the present invention is applied to a TOC meter will be described with reference to the attached drawings. FIG. 1 is a configuration diagram showing the TOC meter for on-line measurement using a high-frequency power source UV lamp monitor according to an embodiment of the present invention. FIG. 2 is a diagram showing a configuration of a sample oxidizing portion in the TOC meter of FIG. 1, and FIG. 3A is a diagram showing wiring of a high-frequency power source UV lamp and a high-frequency power source UV lamp monitor included in the sample oxidizing portion.

In the TOC meter in this embodiment, a total carbon (TC) and an inorganic carbon (IC) in a sample solution are measured, and the difference is calculated. That is, the total organic carbon (TOC) concentration can be obtained by the following equation (1).

TOC = TC-IC - - - (1)

As shown in FIG. 1, the TOC meter 10 is provided with a flow path L to which a sample solution is supplied from a sample introduction port 11 for on-line measurement, a sample oxidizing portion 12 provided in the middle of the flow path L, a conductivity cell 13 for detecting the concentration of carbon dioxide by a conductivity sensor, and a liquid feed pump 14, such as, e.g., a tube pump, for feeding a sample solution and passing it through the flow path L.

Hereinafter, for convenience of description, a flow path extending from the sample introduction port 11 to the sample oxidizing portion 12 is defined as a flow path L1, a flow path in the sample oxidizing portion 12 is a flow path L2, a flow path extending from the sample oxidizing portion 12 to the conductivity cell 13 is defined as a flow path L3, and a flow path in the the conductivity cell 13 is defined as a flow path L4.

The sample oxidizing portion 12 has a built-in excimer UV lamp (high-frequency power source UV lamp) 20 connected to a high-frequency power source 19.

As shown in FIG. 2, the excimer UV lamp 20 has a double tube structure, and the inner tube 21 thereof is served as a part of a flow path L (i.e., a flow path L2) for oxidizing a sample solution and delivering it to the conductivity cell 13. A rare gas (e.g., xenon) is sealed in the outer tube 22. Electrodes 22c and 22d are provided on the inner wall 22a and the outer wall 22b of the outer tube 22, respectively, and the electrodes 22c and 22d are connected to a high-frequency power source 19 of 10kHz to 1,000 kHz via a feeder 23. For example, it is configured such that when a high-frequency power of 100 kHz is applied from the high-frequency power source 19, ultraviolet rays generated by dielectric barrier discharge are irradiated toward the inner tube 21.

The excimer UV lamp 20 is accommodated in a hermetically sealed container 24 so that ultraviolet rays and air containing harmful ozone do not leak from the inside of the hermetically sealed container 24 to the outside.

Further, a detection circuit 30 for performing lighting confirmation is attached to a portion located outside the hermetically sealed container 24 in the feeder 23 connecting the high-frequency power source 19 and the excimer UV lamp 20. The detection circuit 30 is composed of a core member (toroidal core or ferrite core) 31 made of a magnetically permeable material and attached to the outer periphery of the feeder 23 in a ring shape, an induction circuit 32 wound around the core member 31 in a coil shape to extract a high-frequency induction current, and an LED (detection element) 33 connected to the induction circuit 32. The induction circuit 32 is also provided with a rectifying diode 34 and a protective resistor 35 for rectification and protection of the LED 33.

When the measurement is performed using such a TOC meter 10, first, in a state in which the excimer UV lamp 20 is turned off, the sample solution is continuously sucked into the flow path L by the liquid feed pump 14 at a constant flow rate, and the entire flow path L (flow paths L1 to L4) is filled with the sample solution. At this time, since a sample solution not irradiated with ultraviolet rays, that is, a unoxidized sample solution, is flowing through the conductivity cell 13 (flow path L4), the amount of carbon dioxide in the sample solution at this time is detected by the conductivity cell 13 as a signal of inorganic carbon (IC).

Subsequently, the liquid feed pump 14 is stopped and the excimer UV lamp 20 is turned on. In this state, ultraviolet irradiation is performed for about one minute. As a result, the total carbon (TC) of the sample solution staying in the inner tube 21 (flow path L2) of the sample oxidizing portion 12 is oxidized to be converted into carbon dioxide.

Thereafter, when the excimer UV lamp 20 is turned off and suction of the sample solution is resumed at a constant flow rate by the liquid feed pump 14, the sample solution (unoxidized sample solution) in the flow path L3 between the sample oxidizing portion 12 and the conductivity cell 13 flows for a while in the conductivity cell 13, and then the sample solution oxidized in the sample oxidizing portion 12 (flow path L2) reaches the conductivity cell 13. The amount of carbon dioxide at this time is detected as the total carbon (TC).

More specifically, when the sample solution is sucked at a constant flow rate, the time from the completion time T1 of passing the unoxidized sample solution in the flow path L3 to the completion time T2 of passing the sample solution oxidized in the flow path L2 in the conductivity cell is substantially determined by the relationship between the volumes of the flow paths L2 and L3. Therefore, the amount of carbon dioxide is continuously detected with the conductivity cell 13 until at least a period including before and after centering the period from the time T1 to the time T2 elapses, and the peak value of the detection signal of the conductivity cell 13 detected during that period is acquired as a signal of the total carbon (TC).

Then, the total organic carbon (TOC) concentration is calculated from the values of the inorganic carbon (IC) and the total carbon (TC) obtained immediately before based on the equation (1). Thus, the first measurement is completed.

Subsequently, when the sample solution is continuously sucked by the liquid feed pump 14 even after completion of the first measurement, all the flow paths L (L1 to L4) including the conductivity cell 13 are filled with the replaced unoxidized sample solution.

Therefore, by repeating the above measurement flow, it is possible to continuously perform the second and subsequent measurement of the total organic carbon (TOC) concentration.

Next, the lighting confirmation flow of the excimer UV lamp 20, which is a feature of the present invention, will be described.

When measuring the total carbon (TC) concentration, the sample solution is made to be retained in the inner tube 21 (flow path L2) of the sample oxidizing portion 12 and high-frequency power is supplied from the high-frequency power source 19 to excite the excimer UV lamp 20 to start discharge. Thus, by the action of the ultraviolet rays irradiated from the excimer UV lamp 20, an oxidation reaction occurs in the total carbon (TC) in the sample solution, and the total carbon (TC) is converted into carbon dioxide.

However, in cases where ultraviolet rays are not irradiated due to a failure or malfunction of the excimer UV lamp 20, the measurement of the total carbon (TC) concentration is performed without the oxidation reaction being performed, and an erroneous measurement result is shown, so that the user needs to confirm the lighting of the excimer UV lamp 20 using the detection circuit 30 before the measurement.

That is, in the ultraviolet irradiation in the sample oxidizing portion 12, high-frequency energy is supplied from the high-frequency power source 19 to the excimer UV lamp 20 via the feeder 23, so electromagnetic waves are generated around the feeder 23 by the high-frequency energy in the feeder 23. The generated electromagnetic waves are extracted as a high-frequency induction current by the core member 31 attached to the feeder 23 at a position outside the hermetically sealed container 24 and having high magnetic permeability and the induction circuit 32. When the high-frequency induction current flows to the LED 33 via the rectifying diode 34 connected to the induction circuit 32, the LED 33 emits light. Therefore, the lighting of the excimer UV lamp 20 can be confirmed by the lighting of the LED 33.

Although an embodiment of the present invention has been specifically described above, the present invention is not necessarily limited to the above-described embodiment, and it is needless to say that the present invention can be appropriately modified and changed within the scope not departing from the spirit of the present invention. Hereinafter, a modified embodiment thereof will be described.

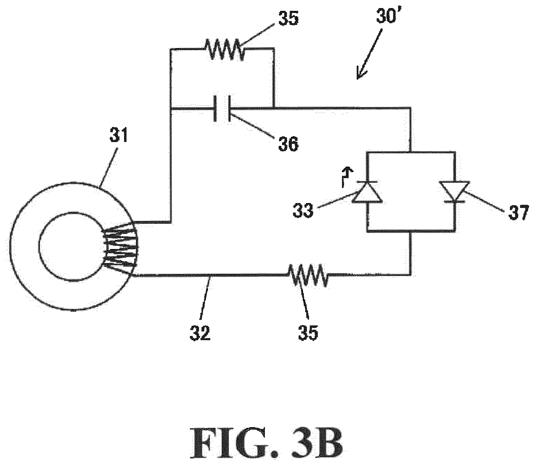

The detection circuit 30 may be any other detection circuit as long as it is a circuit for extracting and detecting an induced current. An example is shown in FIG. 3B. In the detector 30' of FIG. 3B, the reference numeral "35" denotes a protective resistor, the reference numeral "36" denotes a capacitor for AC passage, and the reference numeral "37" denotes a protective diode.

In the embodiment described above, the resistor element may be used as a detection element to detect a voltage signal. In this case, the detected voltage signal may be compared with a threshold voltage set in advance to judge whether or not lighting is on, and when it is equal to or larger than the threshold value, it is judged that lighting is on, and when it is less than the threshold value, it is judged that lighting is off, and the judgment results may be notified by a detection means, such as, e.g., a buzzer.

Further, in the excimer UV lamp 20 used in the above embodiment, since the sample solution passes through the inner tube 21 (flow path L2), it is enough that only the inner tube 21 is irradiated with ultraviolet rays. Therefore, by forming the outer peripheral surface of the outer tube 22 with a material which does not allow transmission of ultraviolet rays, or by adhering or covering the outer tube 22 with a material which does not allow transmission of ultraviolet rays, it is possible to safely use the outer tube 22 without using the hermetically sealed container 24 while eliminating the problems of external irradiation of ultraviolet rays or harmful ozone generation.

### Industrial Applicability

The present invention can be used as a lamp monitor of a UV lamp used in a TOC meter.

### Description of Symbols

- 10:: TOC meter (total organic carbon meter)
- 11:: sample introduction port
- 12:: sample oxidizing portion
- 13:: conductivity cell
- 19:: high-frequency power source
- 20:: excimer UV lamp (high-frequency power source UV lamp)
- 21:: inner tube
- 22:: outer tube
- 22c, 22d:: electrode
- 23:: feeder
- 24:: hermetically sealed housing
- 30:: detection circuit
- 31:: core member
- 32:: induction circuit
- 33:: LED (detection element)
- 34:: rectifying diode
- 35:: protective resistance
- 36:: capacitor
- 37:: protective diode

## Claims

1. A high-frequency power source UV lamp monitor to be attached to a high-frequency power source UV lamp which emits ultraviolet light by supplying high-frequency energy from a high-frequency power source via a feeder, the high-frequency power source UV lamp monitor comprising:
a detection circuit including a core member attached around the feeder in a ring shape and made of a magnetically permeable material, an induction circuit wound around the core member to extract a high-frequency induction current, and a detection element connected to the induction circuit.

2. The high-frequency power source UV lamp monitor as cited in claim 1,
wherein the detection element is an LED.

3. A total organic carbon meter provided with a sample oxidizing portion for oxidizing carbon contained in a sample solution by irradiation with ultraviolet rays to convert the carbon into carbon dioxide,
wherein the sample oxidizing portion uses a high-frequency power source UV lamp for emitting ultraviolet rays by supplying high-frequency energy from a high-frequency power source via a feeder, and is provided with a high-frequency power source UV lamp monitor for confirming lighting of the high-frequency power source UV lamp, and
wherein the high-frequency power source UV lamp monitor comprises a detection circuit including a core member attached around the feeder in a ring shape and made of a magnetically permeable material, an induction circuit wound around the core member to extract a high-frequency induction current, and a detection element connected to the induction circuit.
